# EUROPÄISCHE PATENTANMELDUNG

(11) **EP 2 400 292 A1**
(43) Veröffentlichungstag der Anmeldung: **28.12.2011**
(21) Anmeldenummer: 10006582.0
(22) Anmeldetag: 24.06.2010
(51) Int. Cl.: G01N 21/86, A61B 5/00

(54) **System zur Messung einer Analytkonzentration einer Körperflüssigkeitsprobe**

(71) Anmelder: Roche Diagnostics GmbH, 68305 Mannheim (DE); F.Hoffmann-La Roche AG, 4070 Basel (CH)
(72) Erfinder: Die Erfindernennung liegt noch nicht vor
(74) Vertreter: Mommer, Niels

(57) **Zusammenfassung**

Die Erfindung betrifft ein System zur Messung einer Analytkonzentration in einer Körperflüssigkeitsprobe, mit wenigstens einem Testelement (2) und mit einem Handgerät (1), das ein Gehäuse (4) und einen in einem Innenraum (4a) des Gehäuses (4) angeordneten Sensor (5a) zur Messung der Analytkonzentration einer von einem Testelement (2) aufgenommenen Körperflüssigkeitsprobe aufweist, wobei das Gehäuse (4) den Innenraum (4a) feuchtigkeitsdicht umschließt. Erfindungsgemäß ist vorgesehen, dass das Gehäuse (4) einen für Messsignale durchlässigen Wandabschnitt (4b) aufweist und an dem Gehäuse (4) außerhalb des Innenraums (4a) eine Positioniereinrichtung (6) zum Positionieren eines Testelements (2) an dem für Messsignale durchlässigen Wandabschnitt (4b) angeordnet ist.

## Beschreibung

Die Erfindung geht aus von einem System mit den im Oberbegriff des Anspruchs 1 angegebenen Merkmalen. Ein solches System ist aus der EP 1 881 322 A1 bekannt.

Messsysteme mit einem Handgerät und dazugehörenden Testelementen werden insbesondere zur Messung der Glucosekonzentration schon seit einigen Jahrzehnten von verschiedenen Herstellern angeboten und von Diabetikern weltweit millionenfach verwendet. Testelemente enthalten üblicher Weise Nachweisreagenzien, die bei Kontakt mit einer Körperflüssigkeitsprobe bzw. einem darin enthaltenen Analyten, beispielsweise Glucose, Cholesterin oder Laktat, eine Nachweisreaktion bewirken. Die Stärke der Nachweisreaktion ermöglicht eine Bestimmung der Analytkonzentration und kann beispielsweise photometrisch oder elektrochemisch ausgewertet werden.

Bei dem aus der EP 1 881 322 A1 bekannten System kann das Handgerät geöffnet werden, um Testelementen in einen Geräteinnenraum einzulegen. Das Gehäuse wird anschließend geschlossen und umgibt den Gehäuseinnenraum dann feuchtigkeitsdicht. Das Aufbringen einer Körperflüssigkeitsprobe auf ein Testelement erfolgt über eine Applikationsöffnung des Gerätes, die dafür kurzzeitig geöffnet wird.

Aufgabe der vorliegenden Erfindung ist es, einen Weg aufzuzeigen, wie die Zuverlässigkeit von Systemen zur Messung von Analytkonzentrationen in Körperflüssigkeitsproben erhöht werden kann.

Diese Aufgabe wird erfindungsgemäß durch ein System mit den im Anspruch 1 angegebenen Merkmalen sowie durch ein Handgerät für ein solches System gemäß Anspruch 15 erreicht. Vorteilhafte Weiterbildungen der Erfindung sind Gegenstand von Unteransprüchen.

Bei einem erfindungsgemäßen System weist das Gehäuse des Handgeräts einen für Messsignale durchlässigen Wandabschnitt auf. Beispielsweise kann ein Wandabschnitt transparent sein, so dass Messsignale in Form von Licht durch den Wandabschnitt hindurch treten können. Auf diese Weise können photometrische Messungen mit einem in dem Gehäuse angeordneten Sensor an einem außerhalb angeordneten Testelement durchgeführt werden. Eine weitere Möglichkeit besteht darin, den für Messsignale durchlässigen Wandabschnitt elektrisch leitfähig auszubilden, beispielsweise für elektrochemische Messungen. Bevorzugt werden hierfür einer, besonders bevorzugt zwei oder mehr, Metallkontakte in eine im Übrigen aus Kunststoff ausgebildete Gehäusewand eingebettet.

An dem Gehäuse eines erfindungsgemäßen Handgeräts ist außerhalb des Innenraums eine Positioniereinrichtung zum Positionieren eines Testelements an dem für Messsignale durchlässigen Wandabschnitt angeordnet. Die Positioniereinrichtung kann beispielsweise als ein Anschlag oder eine Aufnahme ausgebildet sein. Ein positioniertes Testelement liegt an dem für Messsignale durchlässigen Wandabschnitt an, so dass mit dem in dem Innenraum des Geräts angeordneten Sensor die Analytkonzentration einer sich außerhalb des Geräteinnenraums befindenden Probe gemessen werden kann. Eine Geräteöffnung zum Einbringen einer Probe oder eines Testelements wird bei einem erfindungsgemäßen Handgerät nicht benötigt. Vorteilhaft kann deshalb eine Beeinträchtigung eines erfindungsgemäßen Handgeräts durch Feuchtigkeit oder Staub ausgeschlossen werden, indem der Innenraum des Handgeräts feuchtigkeitsdicht, insbesondere spritzwasserdicht, versiegelt ist.

Ein erfindungsgemäßes Handgerät arbeitet auch in einer feuchten oder staubigen Umgebung zuverlässig, da der Sensor von einem hermetisch verschlossenen Innenraum eingekapselt und deshalb vor schädlichen Umwelteinflüssen geschützt ist. Eindringende Luftfeuchtigkeit oder Wasserdampf kann Messsensoren und andere elektrischen Komponenten beeinträchtigen oder Störsignale erzeugen, welche die Messgenauigkeit herabsetzen. Indem der den Sensor enthaltende Geräteinnenraum gasdicht, insbesondere wasserdampfdicht, verschlossen ist, kann deshalb die Zuverlässigkeit eines Handgeräts erhöht werden.

Ein weiterer Vorteil eines erfindungsgemäßen Handgeräts ist, dass es sich leicht reinigen lässt. Ein Gerät mit einem wasserdichten Gehäuses kann mit abgespült oder mit Desinfektionsmittel gereinigt werden ohne Schaden zu nehmen. Ein Feuchtigkeitsfilm und eine Verschmutzung des für Messsignale durchlässigen Wandabschnitts können zu Messfehlern führen, beispielsweise weil der elektrische Widerstand zwischen zwei in die Wand eingebetteten Kontakten oder die Lichtdurchlässigkeit eines transparenten Wandabschnitts herabgesetzt wird.

Das hermetisch abgedichtete Gehäuse eines erfindungsgemäßen Geräts kann so ausgebildet sein, dass es sich zerstörungsfrei öffnen lässt, beispielweise um einen Austausch oder eine Reparatur von in dem Gehäuseinnenraum enthaltenen Komponenten zu ermöglichen. Bei jedem Öffnen des Geräts besteht aber die Gefahr, dass Feuchtigkeit und/oder Schmutz in den Innenraum gelangt und dort Schaden anrichtet. Bevorzugt ist das Gehäuse deshalb so ausgebildet, dass es sich nur zerstörend öffnen lässt; der Innenraum also hermetisch versiegelt ist. Beispielsweise können zwei Gehäusehälften mit einander verrastet sein und eine dazwischen liegende Dichtung verpressen. Möglich ist es auch, alle Gehäuseteile stoffschlüssig, beispielsweise durch Kleben oder Schweißen, miteinander zu verbinden.

Auf Bedienungselemente kann bei einem erfindungsgemäßen Handgerät verzichtet werden, indem die Positioniereinheit mit einem Detektor ausgestattet wird, der die Anwesenheit eines Testelements erfasst und dann das Handgerät aktiviert. Der Detektor kann beispielsweise ein mechanischer Schalter, insbesondere ein Mikroschalter, ein Berührungssensor, insbesondere ein kapazitiver oder induktiver Berührungssensor, sein. Das Handgerät kann aber auch mit Bedienungselementen, beispielsweise einer Taste, versehen sein, durch deren Betätigung es aktiviert werden kann. Tasten verursachen bei einem hermetisch abgedichteten Gehäuse allerdings einen erheblichen Aufwand. Leichter lassen sich Bedienungselemente mit Magnetschaltern, Reedrelais, Hallsensoren, kapazitiven oder optischen Sensoren realisieren. Der Energieverbrauch derartiger Sensoren kann sehr niedrig ausgelegt werden, insbesondere durch Pulsbetrieb.

Eine vorteilhafte Weiterbildung der Erfindung sieht vor, dass in dem hermetisch abgedichteten Innenraum eine Sendeeinheit zur drahtlosem Übertragung von Messergebnissen angeordnet ist. Auf diese Weise kann ein sehr großer Benutzerkomfort erreicht werden, da Messergebnisse an eine Basis- oder Auswerteeinheit übertragen werden können, ohne dass das Handgerät dafür physikalisch mit einem anderen Gerät in Kontakt gebracht werden muss. Die Datenübertragung kann besonders vorteilhaft und energiesparend über das Nahfeld erfolgen. Eine Übertragung von Daten des in dem Geräteinnenraum hermetisch eingeschlossenen Sensors auf ein anderes Gerät kann aber beispielsweise auch über elektrische Kontakte erfolgen, die in einen Wandabschnitt eingebettet sind.

Bevorzugte Sendeeinheit ist ein Transponder. Das Aussenden von Daten wird dann durch Empfang eines Abfragesignals ausgelöst. Bei dem verwendeten Transponder kann es dich um einen aktiven Transponder handeln, also um einen Transponder, der die zum Senden benötigte Energie einem internen Energiespeicher entnimmt. Ein besonders Energie sparender Sendebetrieb kann aber mit einem passiven Transponder erreicht werden. Auf diese Weise ist eine Datenübertragung zwar nur über eher kurze Distanzen möglich, die in der Regel bestenfalls einige Meter betragen, jedoch wird durch die Kommunikation mit einem Abfragegerät, beispielsweise einem Basisgerät, ein interner Energiespeicher des Handgeräts nicht belastet.

Passive Transponder können insbesondere im 13,56 MHz-Band arbeiten. Passive Transpondersysteme können Daten und Energie von einem Lesegerät zum eigentlichen Transpondermodul über das Nahfeld übertragen. Dabei wird im Wesentlichen der magnetische Feldanteil elektromagnetischer Wellen genutzt. Typischerweise ist über das Nahfeld eine Datenübertragung nur über Distanzen von einer oder zwei Wellenlängen möglich, also nur über einige Zentimeter. An sich kann zur Datenübertragung aber auch der sogenannte Freifeldfunk über größere Distanzen, d.h. viele Wellenlängen, genutzt werden. Geeignete System sind beispielsweise Ultra Low Power Bluetooth- oder Gazell-Protokoll.

Um die Vorteile einer komfortablen Datenübertragung nutzen zu können ist ein flüssigkeitsdichter Einschluss des Sensors in einem Geräteinnenraum nicht zwingend erforderlich. Die vorliegende Erfindung betrifft deshalb auch ein System zur Messung einer Analytkonzentration in einer Körperflüssigkeitsprobe, mit wenigstens einem Testelement und mit einem Messgerät, das ein Gehäuse und einen in einem Innenraum des Gehäuses angeordneten Sensor zur Messung der Analytkonzentration einer von einem Testelement aufgenommenen Körperflüssigkeitsprobe aufweist, wobei das Gerät einen Transponder zur drahtlosem Übertragung von Messergebnissen aufweist. Bevorzugt ist der Transponder eines solchen Systems ein passiver Transponder.

Eine weitere vorteilhafte Weiterbildung der Erfindung sieht vor, dass in dem Innenraum des Handgeräts eine Stromquelle angeordnet ist, die wenigstens eine Batterie enthält und geräteinternes Stromnetz speist. Indem eine Batterie ebenso wie der Sensor in dem flüssigkeitsdicht umschlossenen Innenraum angeordnet ist, lässt sich die Zuverlässigkeit des Geräts weiter erhöhen, da die Batterie durch Spritzwasser und ähnliches nicht beeinträchtigt werden kann. An sich ist es aber auch möglich, eine Batterie außerhalb des hermetisch verschlossenen Innenraums anzuordnen, beispielsweise in einem Batteriefach, in dessen Wand elektrische Kontakte eingebettet sind.

Im Rahmen der vorliegenden Anmeldung wird das Wort "Batterie" als Oberbegriff für Primär- und Sekundärzellen verwendet. Während Primärzellen nicht wiederaufgeladen werden können, sind Sekundärzellen wiederaufladbar und werden umgangssprachlich oft als Akkus bezeichnet. Zur Stromversorgung eines erfindungsgemäßen Geräts kann auch eine Kombination aus Primär- und Sekundärzellen verwendet werden.

Insbesondere wenn ein Batteriewechsel nicht vorgesehen ist, ist ein möglichst geringer Energieverbrauch des Handgeräts von großer Bedeutung. Zur Senkung des Energieverbrauchs kann das Gerät einen Schalter aufweisen, mit dem die Stromquelle von dem Stromnetz getrennt werden kann. Auf diese Weise können Leckageströme minimiert werden. Zwischen der Herstellung eines erfindungsgemäßen Geräts und seiner erstmaligen Inbetriebnahme kann im Rahmen üblicher Lieferketten und Lagerhaltung ohne weiteres mehr als ein Jahr liegen. Indem eine eingebaute Batterie in dieser Zeit durch einen geöffneten Schalter von einem geräteinternen Stromnetz abgekoppelt ist, lässt sich die Lebensdauer der Batterie verlängern. Der Schalter kann ein mechanisches Bauteil, das zum Öffnen bzw. Schließen eines Kontakts eine Schaltbewegung ausführt, oder ein elektronisches Bauelement, dessen Leitfähigkeit durch ein Schaltsignal geändert wird, beispielsweise ein Transistorschalter, sein.

Eine weitere Verlängerung der Lebensdauer der Batterie lässt sich dadurch erreichen, dass dieser oder ein anderer Schalter zwischen Messungen geöffnet und so die Stromquelle von dem geräteinternen Strom abgekoppelt wird. Ein entsprechend ausgebildetes Handgerät kann dann zwar keine Uhrzeit und Datumsfunktionen mehr haben, da dafür ein Schaltkreis ständig mit Strom versorgt werden müsste, jedoch kann der Energieverbrauch drastisch reduziert werden, da die interne Stromquelle nur noch während kurzer Zeiten an das Stromnetz angekoppelt ist, während denen das Handgerät in Betrieb ist, beispielsweise für eine Messung oder das Aussenden von Messergebnissen.

Eine weitere vorteilhafte Weiterbildung der Erfindung sieht vor, dass das System ein Basisgerät aufweist, die eine Sende- und Empfangseinheit zur drahtlosen Kommunikation mit dem Handgerät enthält. Das Basisgerät kann beispielsweise durch Aussenden eines Abfragesignals einen Transponder des Handgeräts aktivieren und so in einem Speicher des Handgeräts gespeicherte Messergebnis auslesen. Bevorzugt ist dabei, dass das Handgerät Messergebnisse zusammen mit einer charakteristischen Kennung überträgt. Dies ist insbesondere für einen Einsatz des Systems in Krankenhäusern oder ähnlichen Einrichtungen vorteilhaft. Bei einer solchen Anwendung kann jedem Patienten ein Handgerät zugeteilt werden. Messergebnisse der einzelnen Handgeräte können dann berührungslos mit einem einzigen Basisgerät ausgelesen werden. Anhand der charakteristischen Kennungen können die Messergebnisse dann den einzelnen Handgeräten und somit den Patienten individuell zugeordnet werden.

Die Kombination eines Handgeräts mit einem Basisgerät ermöglicht einen minimalistischen und extrem kostengünstigen Aufbau der Handgeräte. Auswerte- und Anzeigefunktionen können von dem Basisgerät übernommen werden oder bei dem Handgerät nur rudimentär ausgebildet sein. Handgeräte können deshalb mit einer eingekapselten Stromquelle als Wegwerfgeräte ausgebildet sein, die entsorgt werden, sobald die Batterie leer ist. Das Basisgerät kann zum Aufladen der Stromquelle, d.h. der Batterie oder der Batterien, verwendet werden, um die Lebensdauer des Handgeräts zu erhöhen. Eine Aufladung kann über Kontakte, die in eine Gerätewand eingebettet sind, oder beispielsweise durch induktive Kopplung erfolgen. Eine Aufladung der Stromquelle des Handgeräts kann beispielweise auch ermöglicht werden, indem das Handgerät mit einer oder mehreren Solarzellen ausgestattet ist.

Eine drahtlose Kommunikationseinheit eines Handgeräts oder eines Basisgeräts kann beispielsweise auch genutzt werden, um ein Gerät nach der Herstellung mit landesspezifischen Daten zu versehen, beispielsweise Datum- und Uhrzeitdarstellung, Bedienoberflächen oder Displaytexte. Ein Transponder oder eine andere drahtlose Kommunikationseinheit ermöglicht das Einspielen landesspezifischen Daten zu einem beliebigen Zeitpunkt nach der Herstellung und vereinfacht deshalb die Logistik eines Herstellers oder Großhändlers.

Wenn der für Messsignale durchlässige Wandabschnitt transparent ist, beispielsweise um photometrische Messungen zu ermöglichen, kann dieser beschichtet sein.

Beispielsweise kann der transparente Wandabschnitt eine Hartschicht tragen. Insbesondere bei einem transparenten Wandabschnitt aus Kunststoff kann auf diese Weise die Kratzfestigkeit erhöht werden. Kratzer können die Transparenz herabsetzen und optische oder photometrische Messungen beeinträchtigen. Hartschichten können besonders vorteilhaft als Lack aufgetragen werden. Möglich ist auch ein Abscheiden aus der Gasphase, beispielsweise durch Aufdampfen. Geeignete Materialien für Hartschichten sind beispielsweise Polysiloxane.

Alternativ oder zusätzlich kann der transparente Wandabschnitt eine Antireflexschicht tragen. Antireflexschichten sind beispielsweise zum Entspiegeln von Brillengläsern bekannt. Bevorzugt ist eine Antireflexschicht unter einer Hartschicht angeordnet.

Weitere Einzelheiten und Vorteile der Erfindung werden an Ausführungsbeispielen unter Bezugnahme auf die beigefügten Zeichnungen erläutert. Gleiche und aneinander entsprechende Komponenten sind dabei mit übereinstimmenden Bezugszahlen versehen. Es zeigen:
- Figur 1: ein Handgerät zur Messung einer Analytkonzentration in einer Körperflüssigkeitsprobe mit einem Testelement;
- Figur 2: das in Figur 1 dargestellte Handgerät ohne Testelement;
- Figur 3: eine Schnittansicht zu Figur 1;
- Figur 4: ein weiteres Ausführungsbeispiel eines Handgeräts zur Messung einer Analytkonzentration in einer Körperflüssigkeitsprobe;
- Figur 5: eine schematische Schnittansicht des Gehäuseoberteils des in Figur 4 gezeigten Handgeräts;
- Figur 6: eine schematische Schnittansicht des Gehäuseunterteils des in Figur 4 gezeigten Geräts;
- Figur 7: eine Detailansicht zu Figur 5;
- Figur 8: ein weiteres Ausführungsbeispiel eines Systems zur Messung einer Analytkonzentration in einer Körperflüssigkeitsprobe mit einem Handge- rät, einem Basisgerät, einem Testelement und einem Testelementbehälter,
- Figur 9: das Basisgerät des in Figur 8 gezeigten Systems sowie das dazu gehörende Handgerät ohne Testelement.

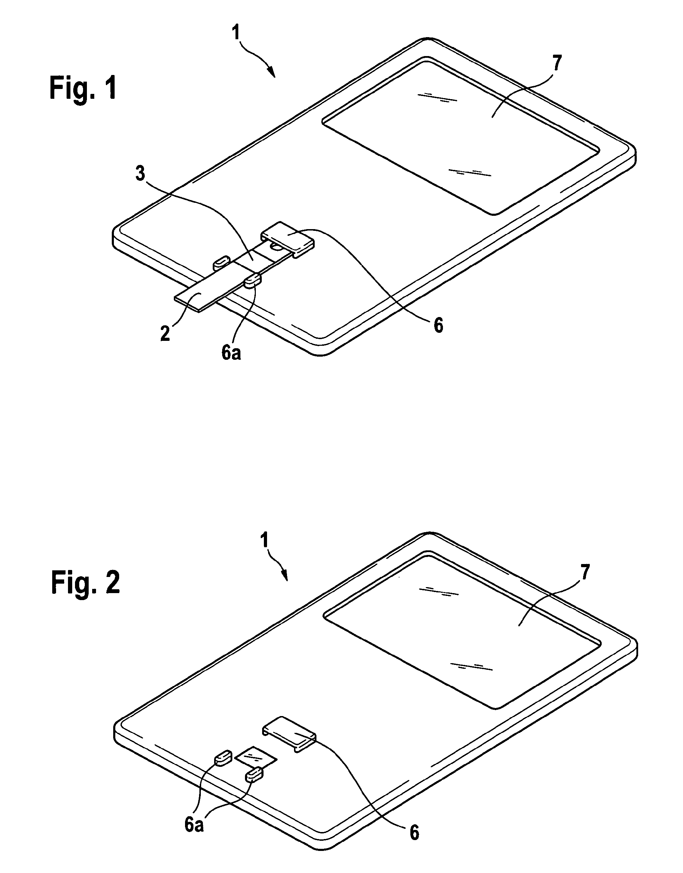

Das in den Figuren 1 bis 3 dargestellte Handgerät 1 bildet zusammen mit einem Testelement 2 ein System zur Messung einer Analytkonzentration in einer Körperflüssigkeitsprobe, beispielsweise zur Messung der Glucosekonzentration oder eines anderen medizinisch bedeutsamen Analyten. Das Testelement 2 ist ein Teststreifen, der ein Testfeld 3 mit Nachweisreagenzien aufweist. Die Nachweisreagenzien bewirken bei Kontakt mit dem Analyten eine Nachweisreaktion, die zu einer Verfärbung des Testfeldes 3 führt. Diese Verfärbung ist umso intensiver, je größer die Analytkonzentration ist. Durch eine fotometrische Messung kann deshalb die Analytkonzentration bestimmt werden. Teststreifen, die nach diesem Prinzip funktionieren, sind von verschiedenen Herstellern handelsüblich erhältlich, so dass sich nähere Erläuterungen erübrigen.

Das Handgerät 1 hat ein Gehäuse 4, das einen Innenraum 4a feuchtigkeitsdicht umschließt. In dem hermetisch abgeschlossenen Innenraum 4a ist ein Sensor 5a zur Messung der Analytkonzentration einer von einem Testelement aufgenommenen Körperflüssigkeitsprobe angeordnet. Der Sensor 5a ist bei dem dargestellten Ausführungsbeispiel Teil einer fotometrischen Messeinrichtung 5, mit welcher der Verfärbungsgrad eines Testfeldes 3 gemessen werden kann. Das Gehäuse 4 hat dafür einen für Messsignale durchlässigen Wandabschnitt, nämlich einen transparenten Wandabschnitt 4b, der als Linse ausgebildet sein kann. Der Wandabschnitt 4b ist aus Kunststoff und an seiner Außenseite von einer Hartschicht aus Polysiloxan bedeckt, die beispielsweise als Lack aufgetragen werden kann. Unter der Hartschicht kann eine Antireflexschicht angeordnet sein.

Die Messeinrichtung 5 enthält zusätzlich zu dem Sensor 5a eine Lichtquelle 5b. Bei dem dargestellten Ausführungsbeispiel wird von der Lichtquelle 5b erzeugtes Licht mit einem Lichtleiter 5c zu dem transparenten Wandabschnitt 4b geführt, nach Durchtritt durch den transparenten Wandabschnitt 4b von dem Testfeld 3 remittiert und dann mit einem weiteren Lichtleiter 5d zu dem Sensor 5a geführt. Die Lichtleiter 5c, 5d haben den Vorteil, die Lichtausbeute zu erhöhen, sind für eine photometrische Messung aber nicht unbedingt erforderlich.

An dem Gehäuse ist 4 außerhalb des wasserdicht verschlossenen Innenraums eine Positioniereinrichtung 6 angeordnet. Mit der Positioniereinrichtung 6 kann ein Testelement so positioniert werden, dass es an dem transparenten Wandabschnitt anliegt und eine Messung zur Konzentrationsbestimmung durchgeführt werden kann. Bei dem dargstellten Ausführungsbeispiel wird die Positioniereinrichtung von einer Tasche und zwei seitlichen Führungselementen 6a gebildet.

In dem abgedichteten Innenraum 4a des Handgeräts 1 ist eine Stromquelle 8, beispielsweise eine Batterie, angeordnet. Die Stromquelle 8 speist ein geräteinternes Stromnetz, über welches die Messeinrichtung 5 mit Strom versorgt wird. Über das geräteinterne Stromnetz können weitere geräteinterne Stromverbraucher, beispielsweise eine eventuell vorhandene Anzeigeeinrichtung 7 mit Strom versorgt werden.

Das Gehäuse 4 kann bei dem dargestellten Ausführungsbeispiel nur durch Zerstörung geöffnet werden. Ein Austausch einer Batterie ist nicht vorgesehen. Um Leckageströme zu minimieren und somit die Lebensdauer der Stromquelle 8 zu erhöhen, enthält das Gerät 1 einen nicht dargestellten Schalter, mit dem die Stromquelle 8 von dem geräteinternen Stromnetz getrennt werden kann. Der Schalter kann beispielsweise ein Mikroschalter sein, der durch Einführen eines Testelements in die Positioniereinrichtung geschlossen wird. Möglich ist es auch, dass der Schalter durch ein dafür vorgesehenes Bedienungselement vom Benutzer geschlossen wird. Für einen möglichst energiesparenden Betrieb ist dabei vorgesehen, dass sich der Schalter selbsttätig öffnet, beispielsweise nachdem eine vorgegebene Zeitspanne verstrichen ist. Die Zeit zum selbsttätigen Öffnen des Schalters kann beispielsweise durch ein RC-Glied oder durch den Takt einer Steuerungseinheit, beispielsweise eine Mikroprozessors oder ASICs, vorgegeben werden.

Die Stromquelle 8 wird bevorzugt so ausgelegt, dass damit in einem Zeitraum von einem Jahr mindestens 500 Konzentrationsbestimmungen durchgeführt werden können. Während bei zum Austausch vorgesehenen Batterien, beispielsweise Knopfzellen, standardisierte Größen und Formen eingehalten werden müssen, bestehen bei fest in einem Gerät eingebauten Batterien große Gestaltungsmöglichkeiten. Eine nicht zum Austausch vorgesehene Batterie kann in nahezu beliebiger Form konzipiert werden, beispielsweise als verformbarer Beutel, und deshalb Geräteinnenraum optimal ausnutzen, so dass ein vorteilhaft kompaktes Handgerät möglich ist.

Um eine umweltfreundliche Entsorgung der in dem wasserdichten Innenraum 4a angeordneten Batterie sowie der Elektronikkomponenten zu erleichtern, kann das Gehäuse 4 eine Sollbruchstelle 13 haben. Wenn die Stromquelle erschöpft ist oder das Gerät 1 aus sonstigen Gründen nicht mehr brauchbar ist, kann das Gerätegehäuse 4 an der Sollbruchstelle mit geringem Aufwand zerbrochen werden. Dann können die Stromquelle sowie in dem Gehäuseinnenraum 4a enthaltene Elektronikkomponenten entnommen und separat entsorgt werden. Die Sollbruchstelle 13 kann beispielsweise als eine Nut oder sonstige Schwächung einer Gehäusewand ausgebildet sein. Damit eine separate Entsorgung von Batterien nicht nötig ist, können als Stromquelle schweremetallfreie Batterien verwendet, beispielsweise gedruckte Batterien, wie sie von der israelischen Firma Power Paper erhältlich sind.

In dem wasserdicht verschlossenen Gehäuseinnenraum 4a kann eine Sendeeinheit 9 zur drahtlosen Übertragung von Messergebnissen angeordnet sein. Die Sendeeinheit 9 ist bevorzugt ein Transponder, insbesondere ein passiver Transponder. Durch Empfang eines Abfragesignals kann somit das Aussenden von Messergebnissen veranlasst werden. Ein passiver Transponder hat den Vorteil, dass der Sendevorgang die geräteinterne Stromquelle nicht belastet, da die Sendeenergie aus dem Abfragesignal gewonnen wird. Geeignete Transponder sind aus der RFID-Technologie bekannt und in Literatur oft auch unter dem Schlagwort "Near Field Communication" (NFC) beschrieben.

Das Handgerät 1 kann in dem hermetisch verschlossenen Innenraum 4a einen Speicher enthalten, um Messergebnisse abzuspeichern. Messergebnisse können in dem Speicher hintereinander abgelegt werden. Sobald der Speicher voll ist, wird bevorzugt jeweils das älteste Messergebnis überschrieben. Wenn zur Minimierung des Stromverbrauchs die Stromquelle zwischen den Messungen mit einem Schalter von dem geräteinternen Stromnetz abgekoppelt wird, werden Messergebnisse werden in dem Speicher ohne Uhrzeit und ohne Datum abgespeichert. In diesem Fall genügt ein sehr kleiner Speicher. Geräteinterne Uhren lassen sich allerdings mit sehr geringem Stromverbrauch realisieren. Aus therapeutischen Gründen ist es vorteilhaft, Messwerte jeweils mit Uhrzeit und Datum der Messung zu erfassen. Möglich ist es, eine geräteinterne Uhr vorzusehen, die nach der erstmaligen Inbetriebnahme des Geräts ständig an die Stromquelle angeschlossen bleibt oder eine zweite Stromquelle zur Versorgung der geräteinternen Uhr vorzusehen.

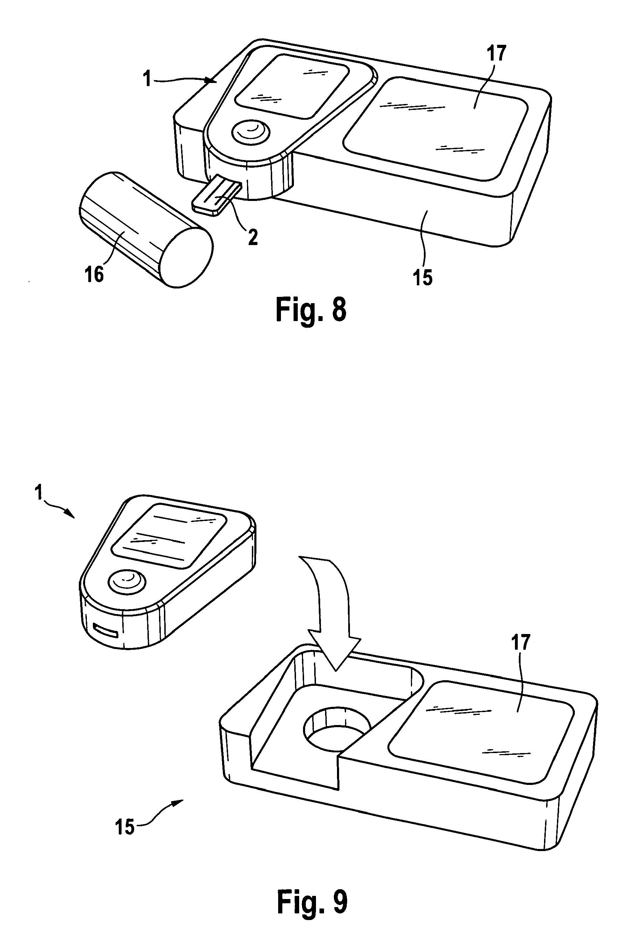

Messergebnisse können mit einem Basisgerät abgefragt werden. Ein Ausführungsbeispiel eines Basisgeräts wird im Zusammenhang mit dem in den Figuren 8 und 9 erläuterten System beschrieben. Ein zu dem Handgerät 1 gehörendes Basisgerät enthält eine Kommunikationseinheit mit einem Sender und einem Empfänger. Durch ein von dem Sender ausgestrahltes Abfragesignal wird das Handgerät veranlasst, eines oder mehrere Messergebnisse zu übertragen. Im einfachsten Fall wird das Handgerät dabei veranlasst, das jeweils aktuellste, d.h. das zu letzt gespeicherte, Messergebnis zu übertragen. Messergebnisse können zusammen mit einer Nummer des Speicherplatzes, in dem sie gespeichert sind, übertragen werden. Das Basisgerät kann anhand dieser Information überprüfen, ob es alle vorhergehenden Messergebnisse vorliegen hat. Fehlende Messergebnisse kann das Basisgerät durch spezielle Abfragesignale gezielt anfordern und das Handgerät zum Aussenden von Messergebnissen veranlassen, die unter dem in einem speziellen Abfragesignal angegebenen Speicherplatz gespeichert sind.

Zusammen mit einem Messergebnis kann beispielsweise auch eine Information über den Status des Handgeräts, beispielsweise Funktions- oder Batteriezustand, übertragen werden.

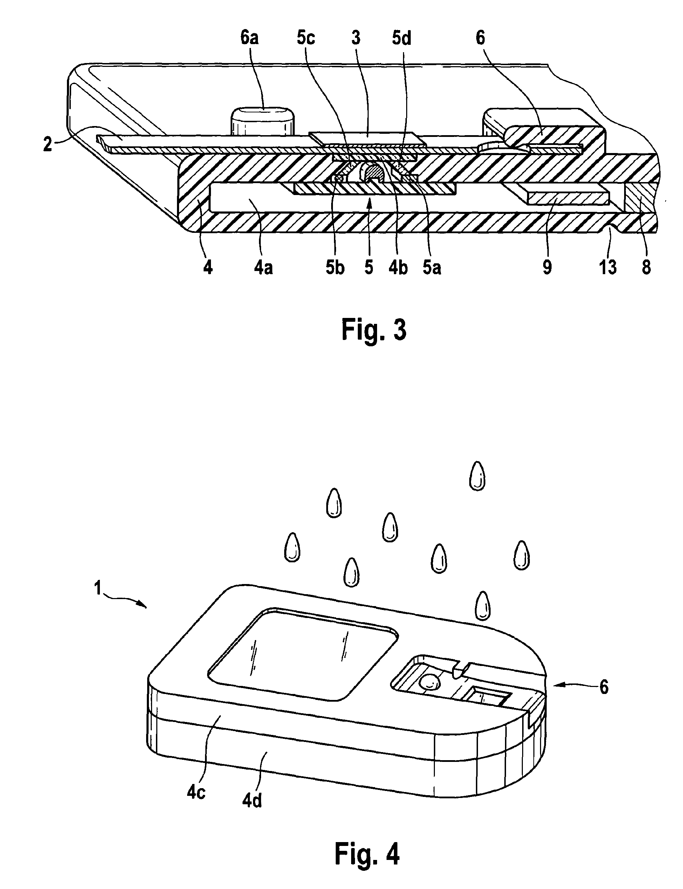

In den Figuren 4 bis 7 ist ein weiteres Ausführungsbeispiel eines Handgeräts 1 zur Messung einer Analytkonzentration in einer Körperflüssigkeitsprobe dargestellt. Nachstehend werden im Wesentlichen nur die Unterschiede zu dem vorstehend beschriebenen Ausführungsbeispiel erläutert. Soweit nicht ausdrücklich anderes angegeben, können Merkmale des Ausführungsbeispiels der Figuren 1 bis 3 auch dem Ausführungsbeispiel der Figuren 4 bis 7 verwirklicht werden.

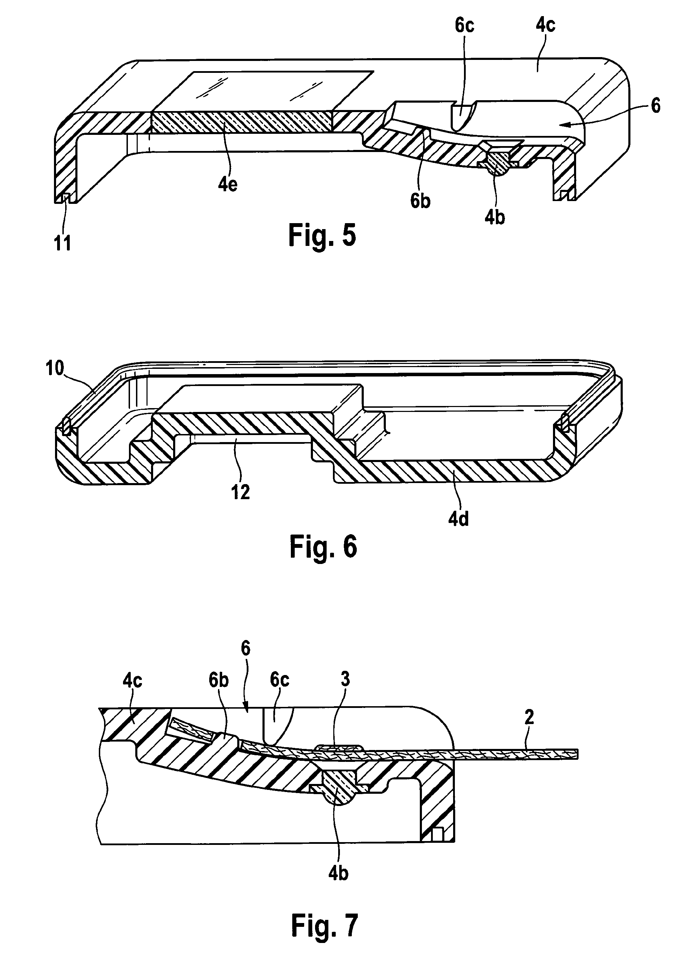

Das in den Figuren 4 bis 7 gezeigte Handgerät 1 hat ein Gehäuse 4, das einen Innenraum 4a spritzwasserdicht umschließt. Das Gehäuse 4 hat einen transparenten Wandabschnitt 4b, der als Linse ausgebildet ist. Mit einer Positioniereinrichtung 6, bei dem dargestellten Ausführungsbeispiel eine Aufnahme, die in dem in Figur 5 dargestellten Gehäuseoberteil 4c ausgebildet ist, kann ein Testelement 2 an dem transparenten Wandabschnitt 4b anliegend positioniert werden. Die Positioniereinrichtung 6 hat bei dem dargestellten Ausführungsbeispiel einen Zapfen 6b, der in ein Loch eines Testelements 2 eingreift, und einen Niederhalter 6c, der einem Abheben des Testelements 2 von dem für Messsignale durchlässigen Wandabschnitt 4b entgegenwirkt.

Das Gehäuse 4 ist aus einem Gehäuseoberteil 4c und einem Gehäuseunterteil 4d zusammengefügt. Das in Figur 5 dargestellte Gehäuseoberteil 4c kann als Zweikomponententeil durch Spritzguss hergestellt werden. Dabei werden transparente Wandabschnitte, beispielsweise die Linse 4b und eine Abdeckung 4e eines Displays aus einem transparenten Kunststoff gespritzt, die übrigen Bereiche des Gehäuses aus einem undurchsichtigen Kunststoff.

Das Gehäuseoberteil 4c wird mit einem Gehäuseunterteil 4d verbunden, beispielsweise durch Verrastung oder Verschraubung. Das Gehäuseunterteil 4d trägt eine umlaufende Dichtung 10 aus einem weichen Kunststoff. Die Dichtung 10 greift beim Zusammenfügen von Gehäuseoberteil 4c und Gehäuseunterteil 4d in eine umlaufende Nut 11 des Gehäuseoberteils 4c ein. Das Gehäuseunterteil 4d kann ebenfalls als Zweikomponentenspritzgussteil hergestellt werden, nämlich aus einer Weichkomponente zur Ausbildung der umlaufenden Dichtung 10 und einer Hartkomponente zur Ausbildung der Gehäusewände.

Bei dem Ausführungsbeispiel der Figuren 4 bis 7 wird eine Batterie als Stromquelle außerhalb des wasserdicht umschlossenen Gehäuseinnenraums, der die Messeinrichtung mit dem Sensor enthält, angeordnet. Das Gehäuseunterteil 4d hat an seiner Unterseite ein Batteriefach 12, das für einen Batteriewechsel geöffnet werden kann. Eine in das Batteriefach 12 eingelegte Batterie ist über in Gehäusewände eingebettete Kontakte an das geräteinterne Stromnetz angeschlossen.

Ebenso wie das vorstehend beschriebene Ausführungsbeispiel kann auch bei dem Ausführungsbeispiel der Figuren 4 bis 7 in dem hermetisch abgeschlossenen Gehäuseinnenraum eine Sendeeinheit, beispielsweise ein Transponder, zur drahtlosen Übertragung von Messergebnissen angeordnet sein.

Die vorstehend beschriebenen Handgeräte können mit einem Basisgerät kombiniert werden, das beispielsweise zusätzliche Auswerte- und Anzeigefunktionen aufweist. In den Figuren 8 und 9 ist ein weiteres Ausführungsbeispiel eines Handgeräts 1 zusammen mit einem dazu passenden Basisgerät 15 dargestellt. Das in Figur 8 dargestellte Handgerät 1 bildet zusammen mit Testelementen 2 ein System zur Messung einer Analytkonzentration in einer Körperflüssigkeitsprobe. Als weitere Komponenten des Systems kommen das Basisgerät 15 und ein Testelementbehälter 16 hinzu.

Das Handgerät 1 kann ebenso wie die vorstehend beschriebenen Handgeräte einen wasserdicht verschlossenen Innenraum aufweisen, in dem ein Sensor zur Messung der Analytkonzentration einer von einem Testelement aufgenommenen Körperflüssigkeitsprobe angeordnet ist, wobei das Gehäuse einen für Messsignale durchlässigen Wandabschnitt, beispielsweise einen transparenten Wandabschnitt, aufweist. Die Positioniereinrichtung 6 zum Positionieren eines Testelements 2 an dem für Messsignale durchlässigen Wandabschnitt ist bei dem dargestellten Ausführungsbeispiel als ein Schlitz bzw. eine Tasche an einer Stirnseite des Geräts 1 ausgebildet.

Eine Stromquelle in Form von einer oder mehreren Batterien kann in dem wasserdicht verschlossenen Innenraum oder ähnlich wie bei dem Ausführungsbeispiel der Figuren 4 bis 7 in einem Batteriefach außerhalb des wasserdicht verschlossenen Innenraums angeordnet sein. Im Unterschied zu den vorstehend beschriebenen Ausführungsbeispielen hat das in den Figuren 8 und 9 dargestellte Handgerät 1 aber keine Anzeigeeinrichtung. Messergebnisse werden von dem Handgerät 1 drahtlos, beispielsweise mit einem Transponder, an das Basisgerät 15 übertragen. Wie in den Figuren 8 und 9 dargestellt, kann das Basisgerät 15 kann eine Aufnahme aufweisen, in die das Hangerät 1 eingelegt werden kann.

Das Basisgerät 15 hat eine Anzeigeeinrichtung 17, beispielsweise eine Flüssigkristallanzeige, mit der Messergebnisse angezeigt werden können. Das Basisgerät 15 kann ferner eine Auswerteeinheit, beispielsweise ein Mikroprozessor oder ein Mikroprozessorsystem mit Prozessor, Speicher, I/O-Port, Taktgenerator etc., enthalten, um Messergebnisse auszuwerten und somit Informationen über den Gesundheitsstatus bzw. den Behandlungsfortschritt eines Patienten zu ermitteln. Weitere Funktionen des Basisgeräts 15 können auf einer internen Uhr des Basisgeräts beruhen, etwa um einen Benutzer durch ein optisches oder akustisches Signal an eine Messung zu erinnern oder auf kritische Messergebnisse hinzuweisen. Das Basisgerät 15 kann mit einer Messaging-Funktion versehen sein, beispielsweise um Messergebnisse, Daten über den Behandlungsfortschritt oder Alarmsignale zu übermitteln, beispielsweise per SMS. Das Basisgerät 15 kann hierfür eine Bluetooth-Schnittstelle oder die SMS-Basisfunktionalität eines Mobiltelefons aufweisen. Möglich ist es auch, das Basisgerät 15 über eine Schnittstelle mit einem Host-Computer kommunizieren zu lassen. Beispielsweise kann das Basisgerät 15 eine USB Schnittstelle aufweisen.

Das Basisgerät 15 kann für eine manuelle Eingabe von Daten vorgesehen sein, beispielsweise indem es eine Tastatur, einen Sensorbildschirm (häufig als Touchscreen bezeichnet), oder sonstige Eingabeelemente aufweist. Wenn Messergebnisse von einem Handgerät 1, wie im Zusammenhang mit dem Ausführungsbeispiel der Figuren 1 bis 3 beschrieben, ohne Zeitangabe übertragen werden, kann die zu einem Messergebnis gehörende Zeitangabe beispielsweise manuell in das Basisgerät eingegeben werden. Dabei besteht die Möglichkeit, Messergebnissen zunächst jeweils den Zeitpunkt der Datenübertragung zuzuordnen, so dass ein Benutzer nur bei einer längeren Trennung von dem Basisgerät Zeitpunkte eingeben bzw. korrigieren muss. Beispielsweise kann ein Benutzer das kompakte Handgerät 1 mit sich führen, aber das größere Basisgerät in seiner Wohnung lassen. Nur falls ein Benutzer Messungen außerhalb seiner Wohnung vorgenommen hat, weichen die Zeitpunkte der Datenübertragung dann von den Zeitpunkten der Messungen ab, so dass eine manuelle Eingabe angebracht sein kann.

Bei dem dargestellten System kann der Testelementbehälter 16 zusätzlich zu mehreren Testelementen 2 auch einen Transponder enthalten, der durch ein Abfragesignal zum Aussenden von Daten veranlasst werden kann. Der Transponder des Testelementbehälters 16 beispielsweise Kalibrationsdaten zur Verfügung stellen, welche die in dem Testelementbehälter 16 enthaltenen Testelemente 2 charakterisieren. Eine Kommunikationseinheit des Basisgeräts 15 kann durch Aussenden eines Abfragesignals den Transponder des Testelementbehälters 16 zur Datenübertragung veranlassen und sich auf diese Weise benötigte Kalibrationsdaten oder ähnliches beschaffen. In entsprechender Weise kann sich das Basisgerät 15 Messergebnisse von dem Handgerät 1 besorgen, nämlich durch Aussenden eines Abfragesignals.

Mit dem Transponder des Testelementbehälters 16 können zusätzlich auch weitere Informationen, beispielsweise aktuelle Produkt- oder Marketinginformationen zur Verfügung gestellt werden. Diese Informationen können einem Benutzer mit der Anzeigeeinrichtung 17 des Basisgeräts 15 angezeigt werden. Vorteilhaft können derartige Informationen nicht nur vom Hersteller, sondern beispielsweise auch von Zwischenhändlern wie Apotheken oder ähnlichem in einem Speicher des Transponders des Testelementbehälters 16 abgelegt werden. Daneben besteht auch die Möglichkeit, dass das Basisgerät 15 Informationen in einen beschreibbaren Speicher des Testelementbehälters 16 schreibt. Im Rahmen eines Pfandsystems lässt sich erreichen, dass Testelementbehälter von Benutzern wieder zurückgegeben werden und von dem Basisgerät 15 in den Speicher geschriebene Informationen somit zum Hersteller des Systems gelangen. Auf diese Weise kann ein Hersteller Informationen über das Benutzerverhalten, beispielsweise über die Häufigkeit von Messungen oder ähnlichem erhalten.

Ein Speicher des Testelementbehälters 16 kann an sich auch über elektrische Kontakte beschrieben werden. Eine drahtlose Kommunikation hat aber den Vorteil eines größeren Benutzerkomforts. Beispielsweise kann der Speicher des Testelementbehälters 16 als verwendet werden, um die Anzahl der noch unbenutzten oder der bereits benutzen Testelemente 2 abzuspeichern. Dabei können den durchgeführten Messungen korrespondierende Daten gespeichert werden, beispielsweise Temperaturen, Betriebsspannungen Messwerte und ähnliches.

Das Basisgerät hat eine eigenständige Stromversorgung, beispielsweise durch Batterien und/oder einen Netzanschluss.

Die Vorteile einer komfortablen Datenübertragung über einen Transponder des Handgeräts 1 können auch bei einem Handgerät verwendet werden, dessen Innenraum nicht feuchtigkeitsdicht verschlossen ist. Auch bei einem solchen Handgerät kann eine fest in das Handgerät 1 eingebaute Batterie, deren Austausch nicht vorgesehen ist, vorteilhaft sein. Die Batterie sollte dabei mindestens 500 Messungen des Handgeräts 1 ermöglichen. Bevorzugt ist die Batterie so bemessen, dass ihre Lebensdauer und damit die Nutzungsdauer des Handgeräts 1 bei mindestens einem Jahr liegt.

### Bezugszahlen

- 1: Handgerät
- 2: Testelement
- 3: Testfeld
- 4: Gehäuse
- 4a: Innenraum
- 4b: Wandabschnitt
- 4c: Gehäuseoberteil
- 4d: Gehäuseunterteil
- 4e: Abdeckung
- 5: Messeinrichtung
- 5a: Sensor
- 5b: Lichtquelle
- 5c: Lichtleiter
- 5b: Lichtleiter
- 6: Positioniereinrichtung
- 6a: Führungselement
- 6b: Zapfen
- 6c: Niederhalter
- 7: Anzeigeeinrichtung
- 8: Stromquelle
- 9: Sendeeinheit
- 10: Dichtung
- 11: Nut
- 12: Batteriefach
- 13: Sollbruchstelle
- 15: Basisgerät
- 16: Testelementbehälter
- 17: Anzeigeeinrichtung/Bedieneinrichtung

## Patentansprüche

1. System zur Messung einer Analytkonzentration in einer Körperflüssigkeitsprobe, mit wenigstens einem Testelement (2) und
mit einem Handgerät (1), das ein Gehäuse (4) und einen in einem Innenraum (4a) des Gehäuses (4) angeordneten Sensor (5a) zur Messung der Analytkonzentration einer von einem Testelement (2) aufgenommenen Körperflüssigkeitsprobe aufweist,
wobei das Gehäuse (4) den Innenraum (4a) feuchtigkeitsdicht umschließt, **dadurch gekennzeichnet, dass**
das Gehäuse (4) einen für Messsignale durchlässigen Wandabschnitt (4b) aufweist und an dem Gehäuse (4) außerhalb des Innenraums (4a) eine Positioniereinrichtung (6) zum Positionieren eines Testelements (2) an dem für Messsignale durchlässigen Wandabschnitt (4b) angeordnet ist.

2. System nach Anspruch 1, **dadurch gekennzeichnet, dass** der für Messsignale durchlässige Wandabschnitt (4b) transparent ist.

3. System nach Anspruch 2, **dadurch gekennzeichnet, dass** der für Messsignale durchlässige Wandabschnitt (4b) als Linse ausgebildet ist.

4. System nach Anspruch 1 oder 2, **dadurch gekennzeichnet, dass** der für Messsignale durchlässige Wandabschnitt (4b) beschichtet ist.

5. System nach einem der vorstehenden Ansprüche, **dadurch gekennzeichnet, dass** in dem Innenraum (4a) eine Stromquelle (8) angeordnet ist, die wenigstens eine Batterie enthält und ein geräteinternes Stromnetz speist.

6. System nach Anspruch 5, **dadurch gekennzeichnet, dass** das Gerät (1) einen Schalter aufweist, mit dem die Stromquelle (8) von dem Stromnetz getrennt werden kann.

7. System nach einem der vorstehenden Ansprüche, **dadurch gekennzeichnet, dass** in dem Innenraum (4a) eine Sendeeinheit (9) zur drahtlosem Übertragung von Messergebnissen angeordnet ist.

8. System nach Anspruch 7, **dadurch gekennzeichnet, dass** die Sendeeinheit (9) ein Transponder ist.

9. System nach einem der vorstehenden Ansprüche, **dadurch gekennzeichnet, dass** das System ein Basisgerät (15) aufweist, das eine Sende- und Empfangseinheit zur drahtlosen Kommunikation mit dem Handgerät (1) enthält.

10. System nach Anspruch 9, **dadurch gekennzeichnet, dass** das Basisgerät (15) eine Anzeigeeinrichtung (7) aufweist.

11. System nach Anspruch 9 oder 10, **dadurch gekennzeichnet, dass** das Basisgerät (15) eine Aufnahme für das Handgerät (1) aufweist.

12. System nach einem der vorstehenden Ansprüche, **dadurch gekennzeichnet, dass** das System einen Testelementbehälter (16) aufweist, der mehrere Testelemente (2) und einen Transponder enthält, der durch ein Abfragesignal zum Aussenden von Daten veranlasst werden kann.

13. System nach Anspruch 12, **dadurch gekennzeichnet, dass** der in dem Testelementbehälter (16) enthaltene Transponder einen beschreibbaren Speicher aufweist, der mit von dem Handgerät (1) oder dem Basisgerät (15) ausgesandten Daten beschreibbar ist.

14. System nach einem der vorstehenden Ansprüche, **dadurch gekennzeichnet, dass** das Gehäuse (4) des Handgeräts (1) eine Sollbruchstelle (13) aufweist.

15. Handgerät zur Messung einer Analytkonzentration in einer Körperflüssigkeitsprobe, mit einem Gehäuse (4) und einen in einem Innenraum (4a) des Gehäuses (4) angeordneten Sensor (5a) zur Messung der Analytkonzentration einer von einem Testelement (2) aufgenommenen Körperflüssigkeitsprobe,
wobei das Gehäuse (4) den Innenraum (4a) feuchtigkeitsdicht umschließt,
**dadurch gekennzeichnet, dass**
das Gehäuse (4) einen für Messsignale durchlässigen Wandabschnitt (4b) aufweist und an dem Gehäuse (4) außerhalb des Innenraums (4a) eine Positioniereinrichtung (6) zum Positionieren eines Testelements (2) an dem für Messsignale durchlässigen Wandabschnitt (4b) angeordnet ist.
